(19) Europäisches Patentamt
European Patent Office
Office européen des brevets

(11) Publication number: **0 091 208**
**B1**

(12) # EUROPEAN PATENT SPECIFICATION

(45) Date of publication of patent specification: **18.06.86**

(51) Int. Cl.[4]: **C 07 C 121/75,**
C 07 C 69/743, A 01 N 53/00

(21) Application number: **83301265.1**

(22) Date of filing: **08.03.83**

(54) **Method for combating insect pests, and novel cyclopropane carboxylates useful as active ingredients therein.**

(30) Priority: **05.04.82 GB 8210006**

(43) Date of publication of application:
**12.10.83 Bulletin 83/41**

(45) Publication of the grant of the patent:
**18.06.86 Bulletin 86/25**

(84) Designated Contracting States:
**AT BE CH DE FR GB IT LI NL SE**

(56) References cited:
**EP-A-0 003 336**
**EP-A-0 008 340**
**EP-A-0 009 709**
**EP-A-0 026 437**
**FR-A-2 442 228**
**FR-A-2 450 249**
**FR-A-2 452 476**
**GB-A-2 054 562**

(73) Proprietor: **IMPERIAL CHEMICAL INDUSTRIES PLC**
**Imperial Chemical House Millbank**
**London SW1P 3JF (GB)**

(72) Inventor: **Punja, Nazim**
**Dunster House 24 Wiltshire Avenue**
**Crowthorne Berkshire (GB)**

(74) Representative: **Bishop, Nigel Douglas et al**
**Imperial Chemical Industries PLC Legal Department: Patents PO Box 6 Bessemer Road**
**Welwyn Garden City Herts, AL7 1HD (GB)**

**EP 0 091 208 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a method of combatting insect ans similar invertebrate pests and to novel cyclopropane derivatives useful as insecticides.

Certain naturally occurring esters of cyclopropane carboxylic acids have long been known to possess insecticidal properties, but these compounds have been too easily degraded by ultra violet light to be of much use in agriculture. Several groups of synthetic compounds based on cyclopropane carboxylic acids (for example those disclosed in British patent specifications nos. 1,243,858 and 1,413,491) have been evaluated in an attempt to discover compounds of sufficient light stability for use as general agricultural insecticides.

A particularly useful group of such compounds is that disclosed in British patent specification no. 2,000,764 and Belgian patent no. 863,151. These compounds combine good light stability with excellent contact and residual insecticidal properties, but, in common with the compounds described in British patent specifications 1,243,858 and 1,413,491, they possess an unsatisfactory toxicity to fish which precludes their use as insecticides in certain situations, e.g. in rice where pisciculture is carried on in the rice paddies. The corresponding fluoro- and bromo-substituted phenoxybenzyl analogues are known from EP—A—0008340 and EP—A—0003336.

The present invention relates to α-Cyano-3-(4-chlorophenoxy)benzyl 3-(2-chloro-3,3,3-trifluoroprop-1-en-1-yl)-2,2-dimethylcyclopropane carboxylate and 3-(4-chlorophenoxy)benzyl *cis*-3-(*Z*-2-chloro-3,3,3-trifluoroprop-1-en-1-yl)-2,2-dimethylcyclopropane carboxylate, which novel compounds possess an extremely high level of insecticidal and acaricidal activity combined with a low toxicity to fish.

The invention moreover provides a method of combatting insect pests at a locus where fish are present or likely to be present which comprises applying to the locus a composition comprising at least one of the novel compounds in an amount which is insecticidally effective but which is substantially non-toxic to the fish.

Particular loci where the method may be practised include for example rice paddies, areas of forest where pesticides run off passes into streams and lakes, and recreational or amenity areas where fish are kept eg, for angling.

It will be appreciated by those skilled in the art that the compounds are capable of existing in various geometrical and stereoisomeric forms. thus there may be *cis* and *trans* isomers arising from the substitution pattern of the cyclopropane ring, and *E*- and Z-isomers arising from the substituted vinyl group. In addition two of the three carbon atoms of the cyclopropane are capable of existing in either R- or S-configurations since they are asymmetrically substituted.

Specific compounds suitable for use in the method of the invention are the compounds of formula (1) set out in Table I wherein the meanings for $R^1$, $R^2$, X and Y and the isomeric composition of each compound is given in the form Z or E (geometry of double bond)- (±), (+) or (−)-*cis* or *trans* (stereochemistry of cyclopropane acid moiety)-(R/S), (S) or (R) (absoluble configuration of carbon bearing group X).

TABLE 1

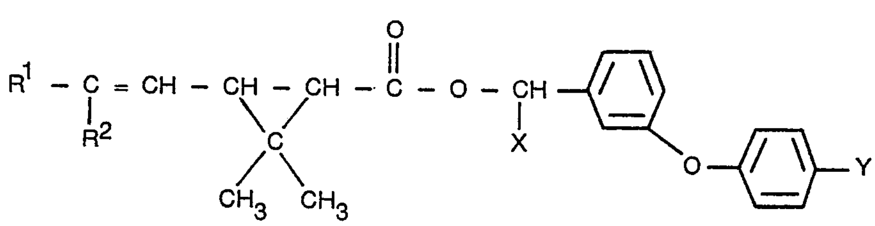

| Compound Number | $R^1$ | $R^2$ | X | Y | Isomeric Composition |
|---|---|---|---|---|---|
| 1 | $CF_3$ | Cl | CN | Cl | Z-(±)-*cis*-(R/S) |
| 2 | $CF_3$ | Cl | H | Cl | Z-(±)-*cis* |
| 3 | $CF_3$ | Cl | CN | Cl | Z-(±)-*trans*-(R/S) |
| 4 | $CF_3$ | Cl | CN | Cl | Z-(±)-*trans*-(R/S) |
| 5 | $CF_3$ | Cl | CN | Cl | Z-(±)--*cis*-(S) |
| 6 | $CF_3$ | Cl | CN | Cl | Z-(±)--*trans*-(S) |

The compounds according to Formula I are esters and may be prepared by conventional esterification processes, of which the following are examples.

(a) An acid of formula:—

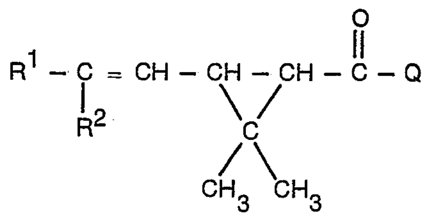

(II)

where Q represents the hydroxy group and $R^1$ and $R^2$ have any of the meanings given hereinabove, may be reacted directly with an alcohol of formula:—

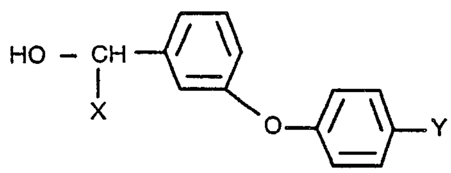

(III)

where X and Y have any of the meanings given hereinabove, the reaction preferably taking place in the presence of an acid catalyst, for example, dry hydrogen chloride.

(b) An acid halide of formula II where Q represents a halogen atom, preferably a chlorine atom, and $R^1$ and $R^2$ have any of the meanings given hereinabove, may be reacted (i) with an alcohol of formula III, the reaction preferably taking place in the presence or a base, for example, pyridine, alkali metal hydroxide or carbonate, or alkali metal alkoxide, or (ii) when X is cyano, with an aldehyde of formula:

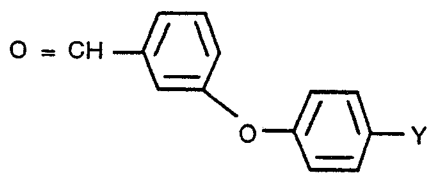

in the presence of an alkali metal cyanide.

(c) An acid of formula II or, preferably, an alkali metal salt thereof, may be reacted with halide of formula:—

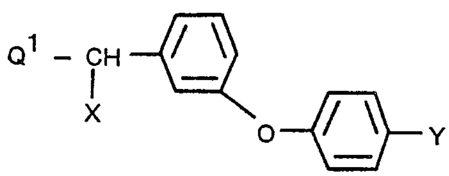

where $Q^1$ represents a halogen atom, preferably the chlorine atom, X, $R^3$, $R^4$ and $R^5$ have any of the meanings given hereinabove, or with the quaternary ammonium salts derived from such halides with tertiary amines, for example pyridine, or trialkyl amines such as triethylamine.

(d) A lower alkyl ester of formula II where Q represents a lower alkoxy group containing up to six carbon atoms, preferably the methoxy or ethoxy group, and $R^1$ and $R^2$ have any of the meanings given hereinabove, is heated with an alcohol of formula III to effect a transesterification reaction. Preferably the process is performed in the presence of a suitable catalyst, for example, an alkali metal alkoxide, such as sodium methoxide, or an alkylated titanium derivative, such as tetramethyl titanate.

All of these conventional processes for the preparation of esters may be carried out using solvents and diluents for the various reactants where appropriate, and may be accelerated or lead to higher yields of product when performed at elevated temperatures or in the presence of appropriate catalysts, for example phase-transfer catalysts.

The preparation of individual isomers may be carried out in the same manner but commencing from the corresponding individual isomers of compounds of formula II. These may be obtained by conventional isomer separation techniques from mixtures of isomers. Thus *cis* and *trans* isomers may be separated by fractional crystallisation of the carboxylic acids or salts thereof, while the various optically active species may be obtained by fractional crystallisation of salts of the acids with optically pure acid.

The optically pure isomeric form of the acid (or its equivalent acid chloride or ester) may then be reacted with the appropriate alcohol to produce a compound of formula I in the form of an individually pure isomer thereof.

The preparation of the compounds of Formula II wherein Q is hydroxy, alkoxy or halo, and $R^1$ and $R^2$ are as defined hereinabove, useful as intermediates in the preparation of the compounds of Formula I, is fully described in British Patent Specification 2,000,764 and in Belgian patent no. 863151.

When the processes for preparing the compounds of Formula I are performed using intermediates which are themselves mixtures of isomers the products obtained will also be mixtures of isomers. Thus the product would be a mixture of (±)-*cis* and (±)-*trans* isomers (perhaps with one form predominating) if the intermediate acid or acid derivative was used in the form of a mixture of (±)-*cis* and (±)-*trans* isomers. If a single isomer, of the acid, e.g. the (+)-*cis* isomer with *Z*-configuration in the 2-chloro-3,3,3-trifluoropropenyl group, was used, the product would also be the single isomer of that stereochemical configuration, or a pair of isomers if there is an asymmetric carbon atom in the alcohol moiety.

The compounds of formula I may be used to combat and control infestations of insect pests and also other invertebrate pests, for example, acarine pests. The insect and acarine pests which may be combatted and controlled by the use of the invention compounds include those pests associated with agriculture (which term includes the growing of crops for food and fibre products, horticulture and aninal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise a insecticide synergiser, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, butylnaphthalene sulphonate, and a mixture of the sodium salts of diisopropyl- and tri-isopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents. Other suitable organic solvents are dimethyl formamide, ethylene dichloride, isopropyl alcohol, propylene glycol and other glycols, isopropyl alcohol, toluene, kerosene, white oil, methylnaphthalene, xylenes and trichloroethylene, N-methyl-2-pyrrolidone and tetrahydro furfuryl alcohol (THFA).

The compositions to be used as sprays may also be in the form of aerosols wherein the formulation is held in a container under pressure in the presence of a propellant such as fluorotrichloromethane or dichlorodifluoromethane.

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 10—85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compositions of the invention are very toxic to wide varieties of insect and other invertebrate pests, including, for example, the following:—

*Aphis fabae* (aphids)
*Megoura viceae* (aphids)
*Aedes aegypti* (mosquitos)
*Dysdercus fasciatus* (capsids)
*Musca domestica* (houseflies)
*Pieris brassicae* (white butterfly, larvae)
*Plutella maculipennis* (diamond back moth, larvae)
*Phaedon cochleariae* (mustard beetle)
*Telarius cinnabarinus* (carmine spider mite)
*Aonidiella* spp. (scale insects)
*Trialeuroides* spp. (white flies)
*Blattella germanica* (cockroaches)
*Spodoptera littoralis* (cotton leaf worm)
*Chortiocetes terminifera* (locusts)
*Diabrotica* spp. (rootworms)
*Nilaparvata lugens* (plant hoppers)
*Nephotettix virescens* (leaf hoppers)
*Chilo suppressalis* (stem borers)
*Tryporyza incurtulos* (stem borers)

The compounds of formula I and compositions comprising them have shown themselves to be particularly useful in controlling pests of rice, for example *Nephotettix* spp. and *Nilaparvata* spp. They are also very useful in combatting insect and acarine pests which infest domestic animals, such as *Lucilia sericata*, and ixodid ticks such as *Boophilus* spp., *Ixodes* spp., *Amblyomma* spp., *Rhipicephalus* spp., and *Dermaceutor* spp. They are effective in combatting both susceptible and resistant strains of these pests in their adult, larval and intermediate stages of growth, and may be applied to the infested host animal by topical, oral or parenteral adminstration.

The following Examples illustrate the various aspects of the invention.

Example 1

This Example illustrates the insecticidal properties of the compounds of Formula I.

The activity of the compound was determined using a variety of insect pests. The compound was used in the form of liquid preparations containing 100, 50, 25, 10 or 12 p.p.m. by weight of the compound. The preparations were made by dissolving the compound in a mixture of solvents consisting of 4 parts by volume of acetone and 1 part by volume of diacetone alcohol. The solutions were then diluted with water containing 0.01% by weight of a wetting agent sold under the trade name "LISSAPOL" NX until the liquid preparations contained the required concentration of the compound. "Lissapol" is a Trade Mark.

The test procedure adopted with regard to each pest was basically the same and comprised supporting a number of the pests on a medium which was usually a host plant or a foodstuff on which the pests feed, and treating either or both the pests and the medium with the preparations. The mortality of the pests was then assessed at periods usually varying from one to three days after the treatment. Details are given in Table IIA.

The results of the tests are given in Table IIB for each of the products A to H at the rate in parts per million given in the second column as a grading of mortality on a scale of 0—9 wherein

0 represents less than 10% mortality
1 represents from 10 to 19% mortality
2 represents from 20 to 29% mortality
3 represents from 30 to 39% mortality
4 represents from 40 to 49% mortality
5 represents from 50 to 59% mortality
6 represents from 60 to 69% mortality
7 represents from 70 to 79% mortality
8 represents from 80 to 89% mortality
9 represents from 90 to 100% mortality

In Table IIB the pest organism used is designated by a letter code and the pest species, the support medium or food, and the type and duration of test is given in Table IIA.

TABLE IIA

| Code Letters (Table IIB) | Pest Species | Support Medium/Food | Type of Test* | Duration (days) |
|---|---|---|---|---|
| MD | *Musca domestica* (houseflies — adults) | Cotton wool/ milk, sugar | Contact | 1 |
| SL | *Spodoptera littoralis* (cotton leaf worm — larvae) | Cabbage leaves | Contact/ Residual | 3 |
| PX | *Plutella xylostella* (diamond back moth — larvae) | Cabbage leaves | Residual | 3 |
| AF | *Aphis fabae* (aphids) | French beans | Contact | 2 |
| DB | *Diabrotica balteata* (rootworm — larvae) | Filter paper | Contact | 3 |

* "Contact" test indicates that both pests and medium were treated and "residual" indicates that the medium was treated before infestation with the pests.

TABLE IIB

| Compound Number | Rate (ppm) | Pest Species | | | | |
|---|---|---|---|---|---|---|
| | | MD | SL | PX | AF | DB |
| 1 | 100 | 9 | 9 | 9 | 9 | 9 |
| 1 | 50 | 9 | 9 | 9 | 9 | 9 |
| 1 | 25 | 9 | 0 | 7 | 9 | 0 |
| 1 | 12 | 9 | 0 | 2 | 9 | 0 |
| 2 | 100 | 9 | 9 | 9 | 9 | 5 |
| 2 | 10 | 1 | 9 | 0 | 9 | 3 |
| 3 | 100 | 9 | 9 | 9 | 9 | 5 |
| 3 | 10 | 9 | 9 | 8 | 9 | 0 |

Example 2

This Example illustrates the improved safety to fish of the compounds for use in the method of the invention compared with known compounds of similar chemical type. Table III sets out the $LC_{50}$ values for young trout and carp in parts per million for the compounds for use in the method of this invention and parts per billion for the known (reference) compounds.

TABLE III

| Compound No. (Table I) | $LC_{50}$ Trout | $LC_{50}$ Carp |
|---|---|---|
| 1 | 20 ppm | — |
| 2 | 10 ppm | — |
| Cyhalothrin* | 0.73 ppb | 1.34 ppb |
| Cypermethrin** | ca 2 ppb | — |

* (±)-α-cyano-3-phenoxybenzyl (±)-*cis*-3-(Z-2-chloro-3,3,3-trifluoroprop-1-en-1-yl)-2,2-dimethylcyclopropane carboxylate.

** (±)-α-cyano-3-phenoxybenzyl (±)-*cis/trans*-3-(2,2-dichlorovinyl)-2,2-dimethylcyclopropane carboxylate.

Example 3

This Example illustrates the preparation of (±)-α-cyano-3-(4-chlorophenoxy)benzyl (±)-*cis-3-(Z*-2-chloro-3,3,3-trifluoroprop-1-en-1-yl)-2,2-dimethylcyclopropane carboxylate (compound no. 1, Table I).

A. Preparation of (±)-α-cyano-3-(4-chlorophenoxy)benzyl alcohol.

Glacial acetic acid (2.0 ml) was added to a well stirred mixture of 4-chlorophenoxybenzaldehyde (4.65 g), sodium cyanide (1.18 g) and water (10 ml), a temperature being maintained in the range 10—15°C during the addition. After completing the addition the mixture was stirred for a further one hour at this temperature, extracted with methylene chloride and the extracts dried over anhydrous magnesium sulphate. The residual oil (5.0 g) remaining after evaporation of the solvent, which was shown by infra-red spectroscopy to consist principally of the desired product together with a small amount of unreacted aldehyde, was used without further purification in the following Example.

Infra red (liquid film): 3400 (broad), 1585, 1485, 1260 $cm^{-1}$.

(A small peak at 1700 $cm^{-1}$ was also present indicating some unreacted aldehyde).

B. A mixture of (±)-*cis*-3-(*Z*-2-choro-3,3,3-trifluoro-prop-1-en-1-yl)-2,2-dimethylcyclopropane carboxylic acid (1.0 g) was refluxed with excess thionyl chloride to convert it to its acid chloride. The excess thionyl chloride was removed by evaporation under reduced pressure and the residue freed from the final traces by azeotropic distillation with dry toluene.

The acid chloride was dissolved in dry toluene (10 ml) and mixed with the (±)-α-cyano-3-(4-chlorophenoxy)-benzyl alcohol (1.16 g) obtained in the previous Example. To the stirred mixture at the ambient temperature was added a solution of dry pyridine (0.5 g) in dry toluene (2.0 ml), resulting in the formation of a white precipitate. After stirring for one hour the mixture was diluted with more toluene and then washed with water, and with saturated aqueous sodium bicarbonate solution, and dried over anhydrous magnesioum sulphate. After filtering to remove the solid matter the filtrate was concentrated by removal of the solvent by evaporation under reduced pressure and colourless residual oil (2.2 g) was purified by wet column chromatography (silica gel column, chloroform eluent) to yield pure (±)-α-3-(4-chlorophenoxy)benzyl (±)-*cis*-3-(*Z*-2-chloro-3,3,3-trifluoroprop-1-en-1-yl)-2,2-dimethylcyclopropane carboxylate (1.2 g) as a colourless oil.

N.m.r. ($CDCl_3$) ppm:1.15—1.40 (m,6H); 1.92—2.37 (m,2H); 6.27 (d,1H); 6.67—7.46 (m,9H).

Infra red (liquid film): 3075, 2950, 1740, 1650, 1585, 1485, 1300, 1270, 1250, 1160, 1130, 1075, 1000, 950, 760 $cm^1$.

Example 4

Other compounds useful in the method of the invention may be prepared by the procedures of Example 3 from the appropriate reactants as follows:

(a) (±)-α-cyano-3-(4-chlorophenoxy)benzyl (±)-*trans*-3-(*Z*-2-chloro-3,3,3-trifluoroprop-1-en-yl)-2,2-dimethylcyclopropane carboxylate (compound no. 3, Table I).

$^1H$ n.m.r.($CDCl_3$)ppm: 1.24—1.42(m,6H); 1.82—2.64(m,2H);0 6.22(d,1H); 6.48(d,1H); 6.98—7.62(m,8H).

Infra red (liquid film): 3060, 2960, 1740, 1650, 1585, 1485, 1280, 1150$cm^{-1}$.

(b) (±)-3-(4-chlorophenoxy)benzyl) (±)-*cis*-3-(*Z*-2-chloro-3,3,3-trifluoroprop-1-en-yl)-2,2-dimethylcyclopropane carboxylate (compound no. 2, Table I).

$^1H$ n.m.r.($CDCl_3$)ppm: 1.30(s,6H); 2.00—2.36(m,2H); 5.12(s,2H); 6.90—7.50(m,9H);

Infra red (liquid film): 3070, 2960, 1725, 1650, 1580, 1480, 1140$cm^{-1}$.

**Claims**

1. α-Cyano-3-(4-chlorophenoxy)benzyl 3-(2-chloro-3,3,3-trifluoroprop-1-en-1-yl)-2,2-dimethylcyclopropane carboxylate.

2. A compound of claim 1 in the form of its *cis* isomer with respect to the substitution pattern of the cyclopropane ring.

3. 3-(4-chlorophenoxy)benzyl *cis*-3-(*Z*-2-chloro-3,3,3-trifluoroprop-1-en-1-yl)-2,2-dimethylcyclopropane carboxylate when substantially free from the corresponding *trans* isomer.

4. A method of combatting insect pests at a locus where fish are present or likely to be present which comprises applying to the locus a composition comprising a compound according to any one of claims 1, 2 and 3, in an amount which is insecticidally effective but which is substantially non-toxic to fish.

5. A method according to claim 4 where the locus is a rice paddy, a forest area where pesticide run off passes into streams, rivers or lakes, or a recreational or amenity area.

**Patentansprüche**

1. 3-(2-Chloro-3,3,3-trifluoroprop-1-en-1-yl)-2,2-dimethylcyclopropancarbonsäure-α-cyano-3-(4-chlorophenoxy)benzyl-ester.

2. Verbindung nach Anspruch 1 in Form ihres cis-Isomers hinsichtlich des Substitutionsschemas des Cyclopropanrings.

3. cis-3-(Z-2-Chloro-3,3,3-trifluoroprop-1-en-1-yl)-2,2-dimethylcyclopropancarbonsäure-3-(4-chlorophenoxy)benzyl-ester, der im wesentlichen frei von dem entsprechenden trans-Isomer ist.

4. Verfahren zur Bekämpfung von Insektenschädlingen an einem Ort, wo es Fische gibt oder wo die Wahrscheinlichkeit besteht, daß es Fische gibt, bei welchem auf den Ort eine Zusammensetzung aufgebracht wird, die eine Verbindung nach einem der Ansprüche 1, 2 oder 3 in einer solchen Menge enthält, die insektizid wirksam ist, die aber gegenüber den Fischen im wesentlichen ungiftig ist.

5. Verfahren nach Anspruch 4, bei welchem der Ort eine Reisanpflanzung, ein Waldgebiet, von welchem ablaufendes Pestizid in Bäche, Flüsse oder Seen gelangt, oder ein Erholungs- oder Freizeitgebiet ist.

**Revendications**

1. Le 3-(2-chloro-3,3,3-trifluoroprop-1-ène-1-yl)-2,2-diméthylcyclopropane-carboxylate d'α-cyano-3-(4-chlorophénoxy)benzyle.

2. Composé suivant la revendiocation 1, sous la forme de son isomère cis par rapport au modèle de substitution du noyau de cyclopropane.

3. Le *cis*-3-(*Z*-2-chloro-3,3,3-trifluoroprop-1-ène-1-yl)-2,2-diméthylcyclopropane-carboxylate de 3-(4-chlorophénoxy)benzyle lorsqu'il est principalement dépourvu de l'isomère *trans* correspondant.

4. Procédé pour combattre des insectes parasites en un lieu où des poissons sont présents ou susceptibles d'être présents, qui consiste à appliquer en ce lieu une composition comprenant un composé suivant l'une quelconque des revendications 1, 2 et 3 en une quantité qui produit un effet insecticide mais qui est principalement non toxique envers les poissons.

5. Procédé suivant la revendication 1, dans lequel le lieu est une rizière, une zone forestière où l'eau de ruissellement chargée de pesticide passe dans des fleures, des rivières ou des lacs, ou une aire de récréation ou de loisir.